Europäisches Patentamt

European Patent Office

Office européen des brevets

(11) Publication number: **0 212 287**
**A2**

## (12) EUROPEAN PATENT APPLICATION

(21) Application number: 86110002.2

(22) Date of filing: 21.07.86

(51) Int. Cl.⁴: **C07C 85/20** , **C07C 87/20**

(30) Priority: 25.07.85 JP 162945/85

(43) Date of publication of application:
04.03.87 Bulletin 87/10

(84) Designated Contracting States:
**DE FR GB IT NL SE**

(71) Applicant: DAINIPPON INK AND CHEMICALS, INC.
35-58, Sakashita 3-chome
Itabashi-ku, Tokyo 174(JP)

(72) Inventor: Asada, Tohru
4-18-6, Chuodai Shisui-machi
Inba-gun Chiba-ken(JP)
Inventor: Miura, Yasuhisa
3010, Oaza Yanagawa Hazaki-machi
Kashima-gun Ibaraki-ken(JP)
Inventor: Kataoka, Yasuki
3-3-379, Higashishisui Shisui-machi
Inba-gun Chiba-ken(JP)

(74) Representative: Kraus, Walter, Dr. et al
Patentanwälte Kraus, Weisert & Partner
Thomas-Wimmer-Ring 15
D-8000 München 22(DE)

(54) Process for producing aliphatic diamine dimers.

(57) A process for producing aliphatic diamine dimers, which comprises heating an aliphatic diamine represented by the general formula

$H_2NRNH_2$

wherein R represents an aliphatic group having 7 to 14 carbon atoms,

in the presence of at least one catalyst selected from the group consisting of palladium, platinum, rhodium and ruthenium to deammoniate it.

This invention relates to a process for producing dimers (to be referred to as triamines) from aliphatic diamines (to be referred to simply as diamines) by a deammoniation reaction.

Triamines are used as materials for dispersing agents, polymeric modifiers and intermediates for the synthesis of medicines and agricultural chemicals. For example, 1,17-diamino-9-azaheptadecane obtained from 1,8-diaminooctane is useful as an intermediate for the synthesis of an agricultural-horticultural fungicide, iminoctadine.

For the production of triamines, a method developed by the present inventors (Japanese Patent Publication No. 8381/1983) has been known which comprises deammoniating a diamine in the presence of a liquid acid catalyst such as nitric acid.

In the reaction of forming a triamine from a diamine, the resulting triamine further undergoes deammoniation to form a by-product polyamine other than the desired product. The ratio of the polyamine formed increases as the conversion of the reaction (the percentage of the amount of ammonia actually formed based on the theoretical amount of ammonia formed when it is assumed that all the diamine is converted to the triamine) increases. The yield of the triamine becomes highest when the conversion based on the amount of the diamine charged is about 65%, and decreases at higher conversions. But when the unreacted diamine is recovered, the yield of the triamine becomes higher as the conversion based on the diamine actually consumed is lower.

Accordingly, from the viewpoint of the triamine producing ability, it is desirable to set the conversion at about 65% so as to maximize the amount of the triamine formed per batch. With the conventional process mentioned above, it is necessary from an economical viewpoint to restrict the conversion to a range of 10 to 40% in order to increase the yield of the triamine based on the consumed diamine.

Furthermore, the conventional process requires complex steps and operations such as mixing and separating of the diamine and the liquid acid catalyst, and the process cannot be operated continuously.

The present inventors have made investigations in order to solve this problem, and have now found that if an aliphatic diamine is heated in the presence of at least one solid catalyst selected from the group consisting of palladium, platinum, rhodium and ruthenium to deammoniate it, a triamine can be obtained efficiently while the formation of the by-product polyamine is inhibited.

The present invention thus provides a process for producing aliphatic diamine dimers, which comprises heating an aliphatic diamine represented by the general formula

$H_2NRNH_2$

wherein R represents an aliphatic group having 7 to 14 carbon atoms,

in the presence of at least one catalyst selected from the group consisting of palladium, platinum, rhodium and ruthenium to deammoniate it.

According to this process of the invention, the conversion can economically be adjusted to 40 to 90%, and the ability to produce the triamine per batch can be strikingly increased. The use of the solid catalyst makes possible a continuous reaction which is difficult with the conventional process described above. Furthermore, since the process of this invention does not require complex steps and operations such as the mixing and separations of the diamine and the liquid acid catalyst as in the conventional process, triamines can be produced with higher efficiencies.

The aliphatic diamine used in this invention is an aliphatic diamine having 7 to 14 carbon atoms and two primary amino groups indicated by the general formula $H_2NRNH_2$. The aliphatic group may be linear, cyclic or branched. Specific examples of the diamine include 1,7-diaminoheptane, 1,8-diaminooctane, 1,10-diaminodecane, 1,12-diaminododecane, 1,14-diaminotetradecane, 1,5-diamino-2,2-dimethylpentane, 4,4'-methylenebis(cyclohexylamine) and 1,4-bis(aminomethyl)-cyclohexane. Palladium, platinum, rhodium and ruthenium may be used singly or in combination as the catalyst. Preferably, the catalyst is a combination of palladium with at least one of platinum, rhodium and ruthenium. The catalyst may be in the form of a sponge-like or powdery metal, or such a metal deposited on a carrier such as carbon, silica, silica-alumina, alumina, diatomaceous earth, calcium carbonate, zinc carbonate, barium carbonate, barium sulfate, and strontium carbonate

The amount of the catalyst used for a batch system is 0.001 to 10 parts by weight as metal (for example, 0.02 to 200 parts in the case of a 5% palladium-alumina catalyst), preferably 0.005 to 0.5 part by weight, per 100 parts by weight of the diamine. Within this preferred range, the triamine can be produced economically advantageously. The amount of the catalyst in a continuous reaction is not particularly restricted.

The reaction may be carried out in various embodiments. For example, the diamine is directly contacted with the catalyst in the form of powder or pellets. Alternatively, the diamine is contacted with the catalyst in a solvent such as water, methanol, isopropyl ether, benzene and hexane.

The reaction temperature is generally 50 to 250°C, preferably 140 to 200°C. At these preferred temperatures, the reaction time required to attain a conversion of 40 to 80% is 1 to 20 hours, and the rate of the reaction can be easily controlled.

The reaction may be carried out in atmospheric air, or in an atmosphere of nitrogen gas, hydrogen gas, etc. The reaction may be carried out under atmospheric pressure, or if required, under elevated pressures.

The process for producing triamines from diamines in accordance with this invention makes it possible to inhibit the formation of the by-product polyamine and increase the selectivity of the triamine. For example, at a conversion of 65% at which the yield of the triamine based on the amount of the diamine charged is highest, the yield of the triamine in the process of this invention reaches 1.35 times as high as that of the triamine in the conventional process.

Furthermore, the process of this invention does not require the complex steps and operations which are essential in the conventional process. Furthermore, a continuous reaction mode can be easily employed in the process of this invention. According to the continuous reaction mode, the conversion of the reaction can be inhibited by controlling the speed of feeding the starting material and the reaction temperature.

According to the process of this invention, there can be easily obtained 1,17-diamino-9-azaheptadecane useful as a material for iminoctadine, an excellent agricultural-horticultural fungicide, from 1,8-diaminooctane. Accordingly, the process of this invention is useful as a main part of the process for producing iminoctadine.

The following examples illustrate the present invention more specifically. Unless otherwise specified all parts and percentages are by weight.

EXAMPLE 1

A 100 ml three-necked flask was charged with 50 g (0.347 mole) of 1,8-diaminooctane and 1 g of a 5% palladium-alumina catalyst, and fitted with a thermometer, a nitrogen gas introducing tube and an air-cooled condenser. With stirring by a magnetic stirrer, the diamine was reacted in a nitrogen stream at 145 to 150°C. The resulting ammonia was introduced into distilled water put in a beaker from the top of the condenser, and the state of progress of the reaction was monitored by titration with 2N hydrochloric acid. Twelve hours later, when the amount of ammonia formed reached 0.113 mole, the reaction solution was cooled to 60°C. Then, 50 ml of methanol as added to dissolve the amine. The catalyst was separated by suction filtration.

Methanol was evaporated, and the amine was distilled under reduced pressure to give 18.7 g of the diamine at 120 to 140°C/15 mmHg and 17.3 g of a triamine at 165 to 200°C/2 mmHg.

The amount of the polyamine as a distillation residue was 10.8 g.

EXAMPLE 2

By the same procedure as in Example 1, 50 g (0.347 mole) of 1,8-diaminooctane was reacted at 168 to 172°C for 17 hours in the presence of 1 g of a 2.7% platinum-alumina catalyst to form 0.113 mole of ammonia. After the catalyst was separated, the residue was distilled under reduced pressure to give 19.0 g of 1,8-diaminooctane, 17.0 g of a triamine and 10.1 g of a polyamine.

EXAMPLE 3

By the same method as in Example 1, 50 g - (0.347 mole) of 1,8-diaminooctane was reacted at 168 to 172°C for 7.5 hours in the presence of 1 g of a 5% rhodium-aluminum catalyst to form 0.113 mole of ammonia. After the catalyst was separated, the residue was distilled under reduced pressure to give 20.4 g of 1,8-diaminooctane, 16.6 g of a triamine and 9.4 g of a polyamine.

EXAMPLE 4

By the same method as in Example 1, 50 g - (0.347 mole) of 1,8-diaminooctane was reacted at 168 to 172°C for 15 hours in the presence of 1 g of a 5% ruthenium-alumina catalyst to form 0.113 mole of ammonia. After the catalyst was separated, the residue was distilled under reduced pressure to give 19.5 g of 1,8-diaminooctane, 16.0 g of a triamine and 10.5 g of a polyamine.

COMPARATIVE EXAMPLE

A 250 ml four-necked flask was charged with 50 g (0.347 mole) of 1,8-diaminooctane and 35.8 g (0.347 mole) of 1% nitric acid, and fitted with a thermometer, a stirrer, a distilling device and a nitrogen gas introducing tube. In a stream of nitrogen, the contents of the flask were heated to 160°C to distill off water from the flask. Then, the distillation device was replaced by a reflux condenser, and while the temperature of the inside of the flask was maintained at 200 to 205°C. The amount of ammonia formed was monitored by the same method as in Example 1. When the amount of ammonia formed reached 0.113 mole, the reaction solution was cooled to 80°C, and then 83.3 g of a 20% aqueous solution of sodium hydroxide was added. The free amine and inorganic materials were separated and the residue was distilled under reduced pressure. There were obtained 21.9 g of the diamine, 12.8 g of a triamine and 11.7 g of a polyamine.

The results of Examples 1 to 4 and Comparative Example are summarized in Table 1.

| | Catalyst | (A) Amount of the diamine charged | (B) Amount of the triamine formed | (C) Amount of the poly- amine formed | Selectivity of the triamine | | Yield of the triamine | |
|---|---|---|---|---|---|---|---|---|
| | | | | | (*1) | (*3) | (*2) | (*3) |
| Example 1 | 5% Pd alumina | $50^g$ | $17.3^g$ | $10.8^g$ | $61.6\%$ | 118 | $34.6\%$ | 135 |
| Example 2 | 2.7% Pt alumina | 50 | 17.0 | 10.1 | 62.7 | 120 | 34.0 | 133 |
| Example 3 | 5% Rh alumina | 50 | 16.6 | 9.9 | 63.8 | 122 | 33.2 | 130 |
| Example 4 | 5% Ru alumina | 50 | 16.0 | 10.5 | 60.4 | 116 | 32.0 | 125 |
| Comparative Example | nitric acid | 50 | 12.8 | 11.7 | 52.2 | 100 | 25.6 | 100 |

(*1)   Selectivity (%) of the triamine = $\dfrac{(B)}{(B)+(C)}$ x 100

(*2)   Yield of the triamine (%) = $\dfrac{(A)}{(B)}$ x 100

(*3)   Indices calculated when the value obtained in Comparative Example is taken as 100.

EXAMPLE 5

A stainless steel tube with an inside diameter of 2.5 cm was filled with 100 g of 0.5% palladium-alumina pellets which were cylindrical and had a diameter of 3 mm and a length of 3 mm. While the catalyst was heated at 180°C, 1,8-diaminooctane was continuously fed into the tube at a rate of 459 g/hr. The activity of the catalyst became steady 7 hours after initiation of feeding the starting material. For a period of 7 hours from that time. 3127 g of the reaction solution flowed out. The amount of the starting material corresponding to it was 3214 g, and the amount of ammonia formed was 87.2 g. The resulting reaction solution was distilled under reduced pressure to give 1526 g of 1,8-diaminooctane, 1009 g of a triamine and 592 g of a polyamine. The selectivity of the triamine was 63.0%, and its yield was 31.4%.

EXAMPLE 6

By the same method as in Example 1, 50 g (0.384 mole) of 1,7-diaminoheptane was reacted at 145 to 150°C in the presence of 1 g of a 5% palladium-carbon catalyst to form 0.125 mole of ammonia. After the catalyst was separated, the residue was distilled under reduced pressure to give 18.5 g of the diamine, 17.1 g of a triamine and 10.5 g of a polyamine. The selectivity of the triamine was 62.0%, and its yield was 34.2%.

EXAMPLE 7

By the same method as in Example 1, 50 g - (0.250 mole) of 1,12-diaminododecane was reacted at 145 to 150°C in the presence of 0.5 g of a palladium black catalyst to form 0.081 mole of ammonia. After the catalyst was separated, the residue was distilled under reduced pressure to give 17.9 g of the diamine, 18.0 g of a triamine and 11.2 g of a polyamine. The selectivity of the triamine was 61.6%, and its yield was 36.0%.

EXAMPLE 8

By the same method as in Example 1, 50 g - (0.352 mole) of 1,4-bis(aminomethyl)cyclohexane was reacted at 160 to 165°C for 8 hours in the presence of a 5% rhodium/silica-alumina catalyst to form 0.114 mole of ammonia. After the catalyst was separated, the residue was distilled under reduced pressure to give 19.9 g of the diamine, 16.7 g of a triamine and 10.5 g of a polyamine. The selectivity of the triamine was 61.4%, and its yield was 33.4%.

EXAMPLE 9

By the same method as in Example 1, 50 g - (0.290 mole) of 2,4-dimethyl-1,8-octanediamine was reacted at 170 to 175°C for 10 hours in the presence of 1 g of a 5% palladium-barium sulfate catalyst to form 0.094 mole of ammonia. After the catalyst was separated, the residue was distilled under reduced pressure to give 20.8 g of the diamine, 16.5 g of a triamine and 9.8 g of a polyamine. The selectivity of the triamine was 62.7 5, and its yield was 33.0%.

Claims

1. A process for producing aliphatic diamine dimers, which comprises heating an aliphatic diamine represented by the general formula

$H_2NRNH_2$

wherein R represents an aliphatic group having 7 to 14 carbon atoms,

in the presence of at least one catalyst selected from the group consisting of palladium, platinum, rhodium and ruthenium to deammoniate it.

2. The process of claim 1 wherein the catalyst is a combination of palladium with at least one of platinum, rhodium and ruthenium.

3. The process of claim 1 or 2 wherein the catalyst is the metal in spongy or powdery form.

4. The process of claim 1 or 2 wherein the catalyst is supported on a carrier.

5. The process of claim 1 wherein the aliphatic diamine is reacted by continuously passing it through a zone filled with the catalyst.

6. The process of claim 1 or 5 wherein the catalytic metal component is used in an amount of 0.001 to 10 parts by weight per 100 parts by weight of the aliphatic diamine.

7. The process of claim 1 or 5 wherein the reaction is carried out in the presence of a solvent.

8. The process of claim 1 or 5 wherein the reaction is carried out at a temperature of 140 to 200°C for a period of 1 to 20 hours.

The process of claim 1 or 5 wherein the reaction is carried out in an atmosphere of nitrogen gas or hydrogen gas.